Europäisches Patentamt

European Patent Office

Office européen des brevets

(11) Numéro de publication : **0 440 561 A1**

# (12) DEMANDE DE BREVET EUROPEEN

(21) Numéro de dépôt : **91400236.5**

(51) Int. Cl.$^5$ : **C07K 3/24**

(22) Date de dépôt : **31.01.91**

(30) Priorité : 01.02.90 FR 9001195

(43) Date de publication de la demande :
07.08.91 Bulletin 91/32

(84) Etats contractants désignés :
AT BE CH DE DK ES GB GR IT LI LU NL SE

(71) Demandeur : ASSOCIATION POUR LA
RECHERCHE ET LE DEVELOPPEMENT DES
METHODES ET PROCESSUS INDUSTRIELS
(ARMINES)
60, Boulevard Saint-Michel
F-75272 Paris Cédex 06 (FR)

(72) Inventeur : Theveneau, Pascal
4 Allée Philippe Roux
F-93120 La Courneuve (FR)
Inventeur : Carron, Didier
25 ter Rue Paul Déroulède
F-94100 Saint-Maur (FR)
Inventeur : Renon, Henri
10 Avenue Arouet
F-92330 Sceaux (FR)
Inventeur : Roche, Brigitte
48 Rue Bargue
F-75015 Paris (FR)

(74) Mandataire : Bruder, Michel et al
10 rue de la Pépinière
F-75008 Paris (FR)

(54) **Procédé d'extraction et/ou de prépurification sélective de protéines contenues dans des produits lactés.**

(57)    La présente invention concerne des procédés d'extraction et/ou de prépurification sélectives de protéines et composants voisins (tels que glycoprotéines, pseudoprotéines, protéines conjuguées) contenus dans des produits lactés sous toutes formes, y compris le colostrum, faisant appel à au moins une étape de précipitation.

Ces procédés sont caractérisés en ce qu'ils comprennent au moins une dite étape de précipitation par addition d'au moins un acide gras de formule générale $CH_3-(CH_2)_n-COOH$ dans laquelle n est un nombre entier au moins égal à 4, et/ou dérivé et/ou substitué à caractère acide du ou desdits acides, ledit ou lesdits acides gras et/ou dérivés et/ou substitués à caractère acide étant fluides à la température de traitement, et en quantité telle que le rapport initial r entre sa ou leur masse en équivalent acide caprylique, et celle des protéines contenues dans la matière à traiter initialement et que l'on veut précipiter est compris entre 0,5 et 4,5, le pH étant acide, l'étape de précipitation étant suivie d'une étape de séparation du précipité et de la fraction liquide.

FIG.1

EP 0 440 561 A1

# PROCEDE D'EXTRACTION ET/OU DE PREPURIFICATION SELECTIVE DE PROTEINES CONTENUES DANS DES PRODUITS LACTES

La présente invention a pour objet des procédés d'extraction et/ou de prépurification sélectives de protéines contenues dans des produits lactés tels que le lait, le colostrum, le lactosérum ou le sérum du colostrum, que ces produits soient entiers, écrémés, ou sous quelque forme que ce soit et en particulier concentrée ou pulvérulente. Plus particulièrement ces procédés permettent l'extraction sélective d'alphalactalbumine, de bétalactoglobuline et d'immunoglobulines et autres, ainsi que la prépurification, notamment, de protéines mineures. On notera que les compositions des différents produits lactés sont extrêmement différentes de l'un à l'autre et que, par exemple le colostrum, c'est-à-dire le lait des premiers jours après une naissance, est particulièrement riche en certaines protéines et en particulier en immunoglobulines, essentiellement de la classe G.

De nombreuses protéines que l'on peut ainsi extraire présentent des propriétés intéressantes dans de nombreux secteurs économiques. Par exemple l'alphalactalbumine et la bétalactoglobuline (qui fait également partie des albumines) possèdent des propriétés fonctionnelles telles que le pouvoir moussant et émulsifiant ou gélifiant et constituent des additifs alimentaires recherchés. Les propriétés de ces additifs sont d'autant mieux respectées que les protéines sont plus pures. Ces protéines possèdent également, par exemple, un pouvoir nutritionnel et peuvent être utilisées dans la fabrication de laits maternisés. Les propriétés immunisantes des immunoglobulines déjà mentionnées conduisent à des applications en particulier en biotechnologie et dans la pharmacopée vétérinaire.

Il convient également de rappeler qu'une albumine est généralement soluble dans des solutions salines diluées ainsi que dans l'eau et dans une solution saturée de sulfate d'ammonium à 50% et qu'elle n'est pas précipitable dans d'autres solutions aqueuses telles que celles de chlorure de sodium ou de sulfate de magnésium. Cependant une albumine est thermocoagulable.

Les globulines sont solubles dans des solutions salines diluées, mais elles précipitent dans une solution saturée de sulfate d'ammonium à 50% ou encore de sulfate de zinc. L'alphalactalbumine a une mobilité électrophorétique moyenne en solution alcaline ou neutre ; les bétalactoglobulines et l'albumine sérobovine ont une mobilité électrophorétique plus forte et les immunoglobulines une mobilité électrophorétique plus faible. Par ailleurs le lactosérum contient, entre autres, des métalloprotéines telles que la transferrine et la lactoferrine qui est une glycoprotéine fixant deux atomes de fer par molécule. Si dans ce qui suit, on focalisera les applications du présent procédé sur les immunoglobulines, l'alphalactalbumine et la bétalactoglobuline qui représentent des applications particulièrement remarquables de ces procédés, on notera qu'il peut s'appliquer à la prépurification et/ou à l'extraction de la lactoferrine ou éventuellement d'enzymes comme la lactopéroxydase et le lysozyme. On citera également la sérumalbumine et en particulier l'albumine sérobovine ou ASB et les protéoses peptones qui sont des glycoprotéines de faible poids moléculaire (de 4.000 à 22.000) solubles dans l'eau, non thermocoagulables.

Dans le lactosérum de vache, à titre indicatif, on peut donner les concentrations suivantes :

| PROTEINE | CONCENTRATION MOYENNE | POIDS MOLECULAIRE MOYEN |
|---|---|---|
| Immunoglobulines | 0,6 g/l | 140 000 à 960 000 |
| Alphalactalbumine | 1,2 g/l | 13 000 à 15 500 |
| Bétalactoglobuline | 2,8 g/l | 18 000 à 40 000 |
| Sérumalbumine | 0,25 g/l | 60 000 à 80 000 |
| Protéoses Peptones | 0,6 g/l | 4 000 à 22 000 |

Comme précédemment mentionné, les proportions de ces diverses protéines peuvent varier énormément selon le produit de départ ; ainsi la concentration des immunoglobulines dans le colostrum de vache peut dépasser 100 g/l dans les premières heures. Néanmoins les trois premières protéines indiquées au tableau ci-dessus sont généralement majoritaires.

L'extraction sélective de ces différentes protéines est, du fait de leurs applications, une opération industrielle indispensable. De nombreux procédés d'extraction ont déjà été proposés dont certains sont utilisés industriellement.

Un procédé connu est la thermocoagulation qui consiste à former des agrégats de protéines par élévation de température. Cette méthode est simple à mettre en oeuvre mais présente l'inconvénient de dénaturer, au moins partiellement, les protéines. De plus l'alphalactalbumine peut être extraite sélectivement, à condition néanmoins de concentrer et d'acidifier préalablement le produit de départ.

L'ultrafiltration est une autre technique utilisée en extraction de protéines. Elle est particulièrement utilisée dans un but de concentration en protéines solubles mais s'avère très décevante dans sa capacité à séparer sélectivement une protéine. En effet la sélectivité résulte d'une cascade d'ultrafiltrations, de mise en oeuvre assez lourde, qui obère le rendement d'extraction et peut réduire les propriétés spécifiques de la protéine extraite.

Une séparation sélective peut être obtenue par des techniques de chromatographie, notamment par échange d'ions. En faisant passer le produit de départ dans des colonnes appropriées, il est possible d'extraire avec une très bonne pureté, les différentes protéines du lactosérum et du lait. Néanmoins ces procédés sont lourds à mettre en oeuvre et sont coûteux tant en investissement qu'en fonctionnement. La capacité d'adsorption des protéines solubles par les colonnes est de plus très faible, de l'ordre de 100 mg de protéines par gramme d'échangeur. De plus, une haute concentration en lipoprotéines, comme dans le cas du colostrum, conduit à un encrassement des colonnes.

Dans d'autres domaines, par exemple dans l'extraction de protéines du plasma, des méthodes par précipitation sont utilisées. Néanmoins, jusqu'à présent, aucun agent précipitant satisfaisant n'a été trouvé, qui permettrait une extraction sélective économique simple et non nocive, des protéines du lait, du lactosérum, du colostrum ou d'autres produits lactés.

Pour illustrer l'art antérieur dans ce domaine on peut mentionner :

– le document EP-A-0346217 (FONDATION NATIONALE DE LA TRANSFUSION SANGUINE), résultat des travaux de la même équipe de chercheurs que la présente demande, décrit un appareillage et le procédé correspondant d'extraction en continu de protéines. Il s'agit là, en fait, essentiellement d'extraction d'immunoglobuline G et d'albumine, à partir de plasma humain, la précipitation de l'albumine se faisant à pH 4,8 comme l'illustre la description et, en particulier, l'exemple. Cette opération se déroule en une seule étape de précipitation, sans possibilité de fractionnement plus sélectif afin d'extraire d'autres protéines. D'autre part, ce document s'inscrit uniquement dans le cadre d'une mise en oeuvre en continu, selon un protocole assez strict.

– le document EP-A-0338229 (BIOTEST PHARMA GmbH) décrit des extractions d'anticorps à partir du colostrum en 6 étapes, dont une étape principale de précipitation par l'acide chlorhydrique, et pouvant comporter éventuellement une étape complémentaire d'adjonction d'acide caprylique, avant ou après l'étape de stabilisation de la préparation d'anticorps, sixième étape du procédé.

On notera que ces deux documents sont orientés vers les seules immunoglobulines et/ou albumine sans autre sélectivité à l'égard d'autres protéines et se limitent, dans leurs exemples, à des conditions de séparation à pH 4,8.

L'article "Preparation of human immunoglobulin by caprylic acid precipitation" HABEEB & FRANCIS, (PREPARATIVE BIOCHEMISTRY, 14(1), 1-17,1984) souligne que les protéines précipitées dans les conditions de l'art antérieur ne peuvent plus être remises en solution.

L'article "Protein Fractionation by Ammonium Sulphate Rivanol and Caprylic Acid Precipitation" (STEINBUCH) (METHODS OF PLASMA PROTEIN FRACTIONATION, Ed. J.M. CURLING, ACADEMIC PRESS, London 33-56) souligne de son côté l'utilisation de tampon et l'amélioration du rendement par la dilution, technique dont s'écarte la présente invention.

Il s'avère donc que les méthodes d'extraction de protéines par précipitation mentionnant l'utilisation, à un stade quelconque, d'un acide gras tel que l'acide caprylique se sont largement focalisées sur l'extraction d'immunoglobulines G à pH 4,8, et selon des protocoles strictement définis. Des essais effectués par la demanderesse ont montré qu'en étendant largement mais avec précision la gamme des conditions physico-chimiques (pH et stoechiométrie essentiellement), on pouvait élargir considérablement le champ des extractions avec une grande sélectivité et obtenir, à partir d'une même matière première, des concentrés variés de protéines, à haute pureté en une protéine sélectionnée, issus tant de précipités que de surnageants

En conclusion et pour résumer, ces techniques d'extraction de l'art antérieur ne présentent pas simultanément les qualités de simplicité, d'efficacité, de sélectivité, de souplesse et de coût souhaitées.

La présente invention a donc pour but de définir des procédés d'extraction et/ou de prépurification présentant l'ensemble de ces qualités, et faciles à mettre en oeuvre.

La présente invention a pour objet des procédés d'extraction sélective et/ou de prépurification de différentes protéines et composants voisins (glycoprotéines, pseudoprotéines, protéines conjuguées, notamment) contenus dans des produits lactés y compris le colostrum, par précipitation avec des acides gras. Ces procédés sont faciles à mettre en oeuvre et peu coûteux. Comme on le verra par la suite, des procédés plus spécifiques conformes à l'invention permettent l'extraction sélective et/ou la prépurification d'une ou plusieurs protéines, telles que celles citées ci-dessus et en particulier l'extraction sélective des trois protéines majeures à savoir l'alphalactalbumine, la bétalactoglobuline et les immunoglobulines, tout en gardant de préférence le même agent précipitant pour l'extraction et/ou la prépurification de protéines, notamment mineures. Si elles sont extraites grâce aux procédés selon l'invention, les protéines ne sont pas dénaturées et conservent leurs propriétés spécifiques. Par ailleurs aucun prétraitement du produit de départ n'est nécessaire.

Les procédés conformes à la présente invention permettent, entre autres, de traiter un produit lacté qui peut avoir été préalablement concentré par exemple par osmose inverse ou par ultrafiltration, ainsi que mis à l'état deshydraté ou pulvérulent ou lyophilisé ou autre et remis en solution. Le traitement de ces produits, plutôt que celui du lactosérum brut, est particulièrement économique, car il opère sur des volumes largement inférieurs. Il peut également s'agir de tout produit lacté préalablement déminéralisé, délactosé ou délipidé, par exemple. Ils permettent également de traiter le colostrum, ses dérivés et composants.

Plus précisément, la présente invention a pour objet des procédés d'extraction et/ou de prépurification de protéines et composants voisins (notamment glycoprotéines, pseudoprotéines et protéines conjuguées) contenus dans des produits lactés faisant, appel à au moins une étape de précipitation, caractérisés en ce qu'ils comprennent au moins une étape de précipitation par addition d'au moins un acide gras, ledit acide étant de formule générale $CH_3\text{-}(CH_2)_n\text{-}COOH$ dans laquelle n est un nombre entier au moins égal à 4, et/ou au moins l'un de ses (ou leurs) dérivés et/ou substitués à caractère acide, ledit ou lesdits acides gras ou ledit ou lesdits dérivés ou substitués à caractère acide étant fluide (liquide ou visqueux notamment) à la température de traitement, le ou les acides gras, dérivés ou substitués étant en quantité telle que le rapport r entre leur masse et celle des protéines contenues initialement dans la matière à traiter au moment de l'opération et que l'on veut précipiter est compris entre 0,5 et 4,5, le pH étant acide, chaque étape de précipitation étant suivie d'une étape de séparation de la fraction précipitée et de la fraction liquide, généralement surnageante. Le précipité peut être soumis à une resolubilisation réalisée, selon des modes de réalisation préférés de l'invention, soit par élévation du pH, ce qui peut être effectué par apport alcalin, soit par extraction d'acide gras. Cette dernière peut être effectuée par le procédé dit à membrane liquide supportée, tel que décrit dans la demande de brevet français 85.11109 du 19 Juillet 1985, intitulée "Procédé de séparation de protéines et d'acides gras, utilisable dans un procédé de fractionnement industriel de ces protéines".

Elle concerne en particulier un procédé d'extraction d'immunoglobulines caractérisé par le fait que le rapport initial r entre la masse du ou des acides gras (et/ou équivalent en acide caprylique tel que défini ci-après) et celle des protéines à précipiter est de 0,5 à 4,5 et le pH de 3 à 6,5, les immunoglobulines étant récupérées dans la fraction liquide lors d'une étape de séparation.

Dans le même esprit, elle concerne, entre autres, un procédé d'extraction d'un mélange d'alphalactalbumine et de bétalactoglobuline caractérisé par le fait que le rapport initial r entre la masse du ou des acides gras (et/ou équivalent en acide caprylique) et celle des protéines solubles à précipiter est de 0,5 à 4,5 et le pH de 3 à 6,5, l'alphalactalbumine et la bétalactoglobuline étant récupérées, lors d'une étape de séparation, dans la fraction précipitée.

Elle concerne également un procédé d'extraction sélective de l'alphalactalbumine et de la bétalactoglobuline caractérisé par le fait que le rapport initial r, calculé en équivalent acide caprylique, entre la masse du ou des acides gras, et/ou dérivés et/ou substitués à caractère acide, et celle des protéines présentes à précipiter autre que la bétalactaglobuline et les immunoglobulines est compris entre 0,5 et 4,5 et le pH entre 3 et 6,5, une première séparation permettant de récupérer l'alphalactalbumine dans la fraction précipitée, et la bétalactoglobuline et les immunoglobulines dans la fraction liquide, cette fraction liquide étant reprécipitée, puis soumise à une deuxième séparation permettant de récupérer la bétalactoglobuline dans la fraction précipitée.

L'agent précipitant selon l'invention est, comme on l'a mentionné au moins un acide gras ou certains dérivés, ou substitués à caractère acide, l'acide gras étant de formule générale $CH_3\text{-}(CH_2)_n\text{-}COOH$, où n est un nombre entier égal ou supérieur à 4 et, selon un mode de réalisation préféré de l'invention, égal ou inférieur à 10. Il peut s'agir de monoacides tels que ceux du tableau ci-dessous. Il peut également s'agir d'acides à plusieurs fonctions comme par exemple l'acide adipique ou hexanedioïque $HOOC\text{-}(CH_2)_4\text{-}COOH$ ou encore l'acide pimélique ou heptanedioïque $HOOC\text{-}(CH_2)_5\text{-}COOH$. Il peut également s'agir de certains de leurs substitués ou homologues et en particulier d'acides gras monoinsaturés ou diinsaturés, dès lors qu'il est possible de travailler en phase fluide à la température de traitement voulue.

La plupart de ces acides sont disponibles sur le marché à faible coût, ils présentent l'avantage d'être facilement éliminés des solutions finales par exemple par ultrafiltration. Dans le tableau suivant est énumérée la liste des acides préférés conformément à l'invention :

EP 0 440 561 A1

| NOM CHIMIQUE | NOM COURANT | POINT DE FUSION | FORMULE |
|---|---|---|---|
| Hexanoïque | n caproïque | $-3,4\,^{\circ}C$ | $CH_3-(CH_2)_4-COOH$ |
| Heptanoïque | oenanthique | $-7,5\,^{\circ}C$ | $CH_3-(CH_2)_5-COOH$ |
| Octanoïque | caprylique | $+16,7\,^{\circ}C$ | $CH_3-(CH_2)_6-COOH$ |
| Nonanoïque | pélargonique | $+12,5\,^{\circ}C$ | $CH_3-(CH_2)_7-COOH$ |
| Décanoïque | n- caprique | $+31,4\,^{\circ}C$ | $CH_3-(CH_2)_8-COOH$ |
| Undécanoïque | undécylique | $+28,6\,^{\circ}C$ | $CH_3-(CH_2)_9-COOH$ |
| Dodécanoïque | laurique | $+44\,^{\circ}C$ | $CH_3-(CH_2)_{10}-COOH$ |

Le procédé conforme à l'invention fonctionne dans une large gamme de températures, de préférence de 1 à 55°C, ce qui inclut avantageusement la température ambiante, mais on peut également travailler à une température de 4 à 8°C qui est souvent utilisée industriellement pour la conservation des produits. Opérer à température ambiante présente l'avantage de dispenser des frais de refroidissement et de chauffage. Néanmoins l'acide gras utilisé doit être, sinon liquide, au moins fluide à la température choisie. On remarquera à ce sujet qu'il devrait être possible d'utiliser des acides gras dont le coefficient n de la formule générale ci-dessus, est supérieur à 10. Mais la plupart du temps, ces acides ont des points de fusion supérieurs à 55°C qui sont difficilement compatibles avec la présence d'albumine thermocoagulable ; cependant dans les cas où le produit lacté traité contient peu ou pas de composés coagulables, il est possible de travailler avec des acides gras supérieurs tels que l'acide myristique (n=12), l'acide palmitique (n=14), l'acide stéarique (n=16), l'acide arachidique (n=18), l'acide béhénique (n=20) ou encore l'acide lignocérique (n=22), ces acides ayant des températures de fusion comprises entre 58 et 85°C. D'autre part, il serait également possible d'utiliser des acides gras monoinsaturés, tels que par exemple l'acide palmitoléïque ou l'acide oléïque, de points de fusion respectifs : -0,5°C et 13,4°C, ainsi que des acides gras diinsaturés, tels que par exemple l'acide linoléïque ou l'acide arachidonique, de points de fusion respectifs : -5°C et -49,5°C.

Comme indiqué précédemment, les procédés peuvent être mis en oeuvre en utilisant n'importe quel acide gras répondant à la définition donnée, à la 2condition que l'acide soit liquide à la température choisie. Lorsqu'on opère à température ambiante, il est préférable, conformément à l'invention, d'utiliser l'acide caprylique CH3-(CH2)6-COOH de masse moléculaire 144 dont la température de fusion est de 16,7°C. Cet acide est facilement disponible sur le marché. Lorsqu'on opère à température plus basse par exemple de l'ordre de 4 à 8°C, il est préférable d'utiliser l'acide n-caproïque CH3-(CH2)4-COOH de masse moléculaire 116 et dont la température de fusion est de -3,4°C. Cet acide est également facilement disponible sur le marché. Les conditions de précipitation sont définies principalement par le pH du milieu et la quantité d'acide gras. Le pH peut être ajusté, lors des étapes de précipitations et plus généralement lors des diverses opérations, par un simple ajout de base ou d'acide, en utilisant de préférence et respectivement la soude et l'acide acétique facilement disponibles sur le marché et de faible coût. La quantité d'acide gras utilisée est déterminée par le rapport initial r entre la masse d'acide gras, ou dérivé ou substitué à caractère acide et celle des protéines initiales à précipiter.

La séparation des deux fractions peut être effectuée au moyen d'un procédé classique de séparation tel que la microfiltration, la centrifugation, ou l'essorage. La demanderesse préfère néanmoins effectuer une microfiltration à un seuil de coupure de la membrane de l'ordre 0,1 à 1 micromètre et de préférence de 0,45 micromètre.

Une resolubilisation de la fraction précipitée est possible :

La resolubilisation dans les procédés conformes à l'invention peut notamment être réalisée par addition d'un agent permettant d'élever le pH.

Il peut donc s'agir d'une base et l'on utilisera de préférence la soude qui présente les avantages indiqués ci-dessus. On peut également assurer cette resolubilisation par extraction d'acide gras, dérivé ou substitué à caractère acide.

Les procédés conformes à l'invention comprennent un certain nombre d'étapes dépendant du nombre de protéines ou groupes de protéines à extraire et/ou à prépurifier, ces étapes pouvant être éventuellement permutées. Chaque étape comprend essentiellement une précipitation par addition d'acide gras dans un rapport initial r et à un pH, compris dans des gammes dépendant de la ou des protéines à extraire et/ou à prépurifier.

Cette précipitation est suivie par une séparation des deux fractions permettant de récupérer les protéines à extraire et/ou à prépurifier dans la fraction liquide, et/ou dans la fraction précipitée. La fraction précipitée peut également donner lieu à une resolubilisation. Ces étapes peuvent être précédées et/ou suivies d'un ajustement du pH nécessaire, en utilisant de préférence comme cela à été indiqué ci-dessus l'acide acétique ou la soude.

Comme indiqué plus haut, les procédés conformes à l'invention sont compatibles avec plusieurs variantes et permettent donc l'extraction sélective et/ou la prépurification des protéines isolées ou en groupe, et en particulier des trois protéines considérées ci-dessus comme majeures. Les variantes présentent une ou plusieurs précipitations dans des conditions bien spécifiques, les protéines recherchées pouvant se situer soit dans la fraction précipitée, soit dans la fraction liquide.

Pour mieux faire comprendre les caractéristiques techniques et les avantages de la présente invention on va en décrire des exemples de réalisation, étant bien entendu que ceux-ci ne sont pas limitatifs quant à leur mode de mise en oeuvre et aux applications qu'on peut en faire. On se référera aux cinq figures suivantes qui représentent schématiquement et respectivement des diagrammes d'électrophorèse effectuée sur gel d'agarose à haute résolution.

La figure 1 est un diagramme correspondant à l'électrophorèse des protéines d'un lactosérum bovin de départ utilisé dans les deux exemples ci-dessous ;

La figure 2 est un diagramme correspondant aux immunoglobulines obtenues par le procédé selon l'exemple 1 ;

La figure 3 est un diagramme correspondant au mélange d'alphalactalbumine et de bétalactoglobuline obtenu par le procédé selon l'exemple 1 ;

La figure 4 est le diagramme correspondant au mélange enrichi en bétalactoglobuline obtenu par le procédé selon l'exemple 2.

La figure 5 est un diagramme correspondant au mélange enrichi en alphalactalbumine obtenu par le procédé selon l'exemple 2 ;

Les lettres a,b,c,d de toutes ces figures désignent les bandes qui correspondent respectivement à l'albumine sérobovine, à la bétalactoglobuline, à l'alphalactalbumine et aux immunoglobulines.

## EXEMPLE 1.

### Extraction conjointe d'immunoglobulines, d'une part, et d'un mélange bétalactoglobuline alphalactalbumine, d'autre part.

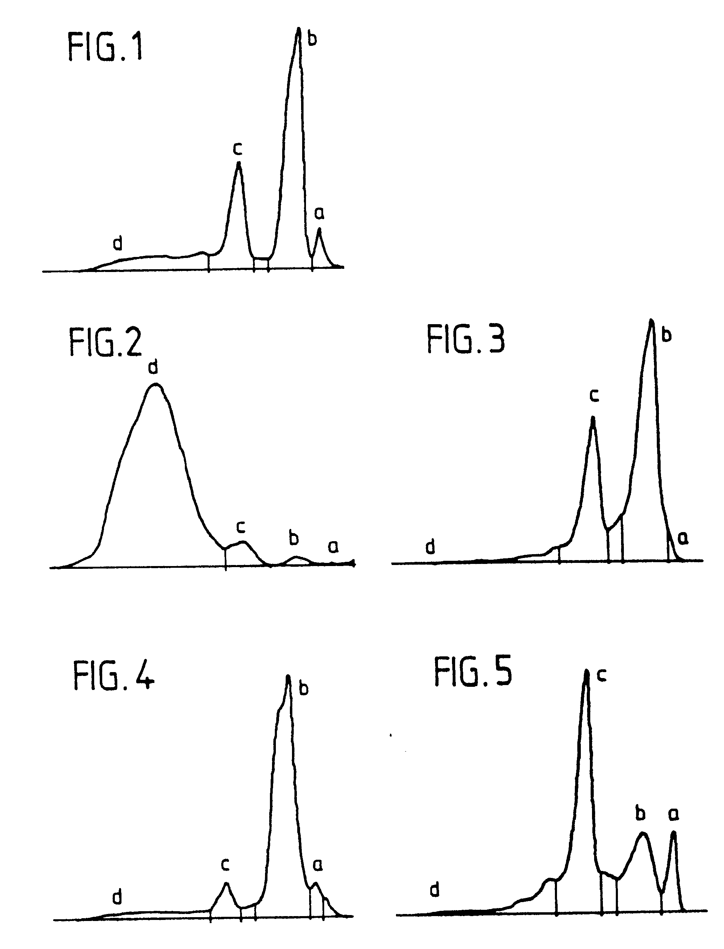

A du lactosérum bovin dont le diagramme d'électrophorèse sur gel d'agarose à haute résolution fait l'objet de la figure 1, on ajoute l'acide caprylique et on ajuste le pH jusqu'à 4,7 ; on travaille de préférence à 20°C considéré comme température ambiante. Le rapport initial r entre la masse d'acide caprylique et celle des protéines présentes dans le lactosérum et que l'on désire précipiter doit être supérieur à 0,5 et de préférence égal à 1,7 et à pH compris entre 3 et 6,5 de préférence de l'ordre de 4,7.

Dans ce qui suit lorsqu'on utilise l'acide caprylique comme acide gras, le rapport initial r est celui entre la masse d'acide caprylique et celle des protéines que l'on veut précipiter à partir du mélange initial. Si l'on utilise un autre acide gras que l'acide caprylique, on calculera le rapport initial r en remplaçant une molécule de monoacide gras par une molécule d'acide caprylique ou une molécule de diacide gras par deux molécules d'acide caprylique. Par exemple, si l'on travaille avec de l'acide caproïque de masse moléculaire 116, on remplacera 116g de ce monoacide par 144g d'acide caprylique dans le calcul de r. Il s'agit donc en fait d'une équivalence entre acide gras et acide caprylique en terme de -COOH. Il en ira de même avec les dérivés et substitués selon le nombre de leurs -COOH.

Dans le cas de cet exemple 1, le rapport initial r étant de 1,7, le pH du mélange est ajusté à 4,7. On obtient ainsi une fraction précipitée que l'on sépare de la fraction liquide par centrifugation. On obtient ainsi, d'une part, des immunoglobulines contenues dans la fraction liquide et, d'autre part, les autres protéines dans la fraction précipitée. On va resolubiliser cette fraction précipitée, par exemple, à l'aide de soude jusqu'à un pH de l'ordre de 7,0. Cette resolubilisation n'est pas sélective. Par un ajustement du pH vers 5,5 à l'aide, par exemple, d'acide acétique, on peut ensuite réaliser une seconde précipitation, permettant, cette fois-ci, de précipiter sélectivement l'alphalactalbumine et la bétalactoglobuline, par l'acide caprylique. Une nouvelle centrifugation permet, d'une part, de séparer la fraction liquide que l'on peut séparer et, d'autre part, le précipité que l'on peut à nouveau resolubiliser, soit par retour à un pH de l'ordre de 7, comme le précédent, par addition d'un agent alcalin, soit par extraction d'acide gras dans les conditions définies dans la demande de brevet citée ci-dessus.

Le précipité resolubilisé est un mélange essentiellement composé d'alphalactalbumine et de bétalactoglobuline dont le diagramme d'électrophorèse fait l'objet de la figure 3. En ce qui concerne la fraction liquide séparée lors de la première centrifugation et qui contient essentiellement des immunoglobulines, son diagramme d'électrophorèse fait l'objet de la figure 2. On constate que, à partir du lactosérum de départ qui contient une quantité importante d'alphalactalbumine (c) et de bétalactoglobuline (b), que l'on retrouve largement au diagramme de la figure 1, les immunoglobulines (d) de la figure 1 sont en quantité négligeable dans le mélange d'alphalactalbumine et de bétalactoglobuline de la figure 3, mais représentent l'essentiel de la fraction liquide (figure 2). L'albumine sérobovine fait l'objet sur ces trois figures de la zone (a) dont on retrouve des traces dans le diagramme du mélange alphalactalbumine, bétalactoglobuline (figure 3).

EXEMPLE 2.

Extraction conjointe d'un mélange enrichi en bétalactoglobuline, d'une part, et d'un mélange enrichi en alphalactalbumine, d'autre part.

Comme à l'exemple précédent on part du même lactosérum (diagramme d'électrophorèse de la figure 1) auquel on va ajouter de l'acide caprylique, en travaillant toujours à température ambiante, avec un rapport initial r de l'ordre 2,8 et l'on ajuste le pH à environ 4,0. Il se forme un précipité et l'on centrifuge l'ensemble pour obtenir, d'une part, la fraction précipitée et, d'autre part, la fraction liquide.

Le précipité est resolubilisé, comme à l'exemple 1, soit en ajustant le pH au voisinage de la neutralité, soit par extraction d'acide gras, de dérivé ou de substitué à caractère acide, et l'on obtient un mélange enrichi en alphalactalbumine dont le diagramme d'électrophorèse fait l'objet de la figure 5. La centrifugation a permis de séparer la fraction liquide qui contient donc essentiellement les immunoglobulines et la bétalactoglobuline. On ajoute à cette fraction liquide de l'acide caprylique dans un rapport initial r de l'ordre de 2,3, et on ajuste le pH à environ 4,7, ce qui entraîne une précipitation. Une deuxième centrifugation permet d'isoler, d'une part, une nouvelle fraction liquide que l'on peut considérer comme non valorisée, tandis que la fraction précipitée séparée, d'autre part, par cette nouvelle centrifugation, est resolubilisé par retour au voisinage de la neutralité ou par extraction d'acide gras, par les moyens mentionnés plus haut, et l'on obtient ainsi un mélange enrichi en bétalactoglobuline dont le diagramme fait l'objet de la figure 4.

Si l'on compare le lactosérum de départ de la figure 1, on constate que pratiquement la totalité de l'alphalactalbumine est passée dans le premier précipité (figure 5) avec des teneurs en bétalactoglobuline et en albumine sérobovine assez faibles, tandis que l'essentiel de la bétalactoglobuline se retrouve dans la fraction précipitée obtenue après la seconde centrifugation (figure 4), le mélange résultant ne présentant que des teneurs extrêmement faibles en albumine sérobovine, en alphalactalbumine et en immunoglobulines.

On notera que l'albumine sérobovine peut être séparée des autres fractions aussi bien à l'exemple 1 qu'à l'exemple 2 par une ou plusieurs ultrafiltrations terminales qui opèrent une sélection en fonction de la masse moléculaire d'une part, et du seuil de coupure de la membrane d'autre part, séparant par exemple, d'un côté, l'alphalactalbumine de faible masse moléculaire et, de l'autre, l'albumine sérobovine de masse moléculaire nettement supérieure. Des cassettes filtrantes, de 5 à 100kD par exemple, permettent de réaliser de telles ultrafiltrations. Une seconde ultrafiltration peut permettre également une concentration en protéines comme, par exemple, l'alphalactalbumine, le ou les acides gras dérivés ou substitués résiduels étant éliminés.

Par ces mêmes procédés, on peut prépurifier et/ou extraire des protéines mineures ou en proportions plus faibles dans le produit de départ. Cela présente l'avantage, par rapport aux techniques actuelles d'éviter un grand nombre d'opérations successives pour parvenir à séparer certains composants difficiles à isoler les uns des autres selon les procédés classiques.

**Revendications**

1. - Procédés d'extraction et/ou de prépurification sélectives de protéines et composants voisins (tels que glycoprotéines, pseudoprotéines, protéines conjuguées) contenus dans des produits lactés sous toutes formes, y compris le colostrum, faisant appel à au moins une étape de précipitation, caractérisés en ce qu'ils comprennent au moins une dite étape de précipitation par addition d'au moins un acide gras de formule générale $CH_3-(CH_2)_n-COOH$ dans laquelle n est un nombre entier au moins égal à 4, et/ou dérivé et/ou substitué à caractère acide du ou desdits acides, ledit ou lesdits acides gras et/ou dérivés et/ou substitués à caractère acide étant fluides à la température de traitement, et en quantité telle que le rapport initial r entre sa ou leur masse en équivalent acide caprylique, et celle des protéines contenues dans la matière à traiter initialement et que l'on veut précipiter est compris entre 0,5 et 4,5, le pH étant acide, l'étape de précipitation étant suivie d'une étape de séparation de la fraction précipitée et de la fraction liquide.

2. - Procédés selon la revendication 1, caractérisés par le fait que la fraction précipitée est resolubilisée.

3. - Procédés selon la revendication 2, caractérisés par le fait que la fraction resolubilisée est reprécipitée, par addition d'au moins un acide gras, dérivé ou substitué à caractère acide.

4. - Procédés selon l'une des revendications 1 à 2, caractérisés par le fait que ladite fraction liquide est soumise à une seconde précipitation par addition d'au moins un acide gras, dérivé ou substitué à caractère acide.

**5.** - Procédés selon l'une des revendications 1 à 4, caractérisés en ce que la resolubilisation est effectuée par élévation de pH réalisée par apport alcalin.

**6.** - Procédés selon l'une des revendications 1 à 5, caractérisés en ce que la resolubilisation est effectuée par extraction d'au moins un acide gras et/ou dérivé et/ou substitué à caractère acide.

**7.** - Procédé d'extraction et/ou de prépurification d'immunoglobulines selon l'une des revendications 1 à 6, caractérisé par le fait que le rapport initial r calculé en équivalent acide caprylique, entre la masse du ou des acides gras et/ou dérivés et/ou substitués à caractère acide, et celle des autres protéines solubles calculé en équivalent acide caprylique et celle des autres protéines solubles présentes est de 0,5 à 4,5 et le pH de 3 à 6,5, les immunoglobulines étant récupérées dans la fraction liquide lors d'une étape de séparation.

**8.** - Procédé d'extraction et/ou de prépurification d'un mélange d'alphalactalbumine et de bétalactoglobuline selon l'une des revendications 1 à 6, caractérisé par le fait que le rapport initial r, calculé en équivalent acide caprylique, entre la masse du ou des acides gras, et/ou dérivés et/ou substitués à caractère acide, et celle des autres protéines présentes est de 0,5 à 4,5 calculé en équivalent acide caprylique, et celle des autres protéines présentes est de 0,5 à 4,5 et le pH de 3 à 6,5, l'alphalactalbumine et la bétalactoglobuline étant récupérées, lors d'une étape de séparation, dans la fraction précipitée.

**9.** - Procédé d'extraction et/ou de prépurification d'alphalactalbumine et de bétalactoglobuline selon l'une des revendications 1 à 6, caractérisé par le fait que le rapport initial r, calculé en équivalent acide caprylique, entre la masse du ou des acides gras, et/ou dérivés et/ou substitués à caractère acide, et celle des protéines présentes à précipiter, calculé en équivalent acide caprylique, et celle des protéines présentes à précipiter autres que la bétalactoglobuline et les immunoglobulines est compris entre 0,5 et 4,5 et le pH entre 3 et 6,5, une première séparation permettant de récupérer l'alphalactalbumine dans la fraction précipitée, et la bétalactoglobuline et les immunoglobulines dans la fraction liquide, cette fraction liquide étant reprécipitée, puis soumise à une deuxième séparation permettant de récupérer la bétalactoglobuline dans la fraction précipitée.

**10.** - Procédés selon l'une des revendications 1 à 9, caractérisés par le fait que la matière traitée préalablement concentrée par osmose inverse ou par ultrafiltration.

**11.** - Procédés selon l'une des revendications 1 à 10, caractérisés par le fait que la matière à traiter est préalablement déminéralisée et/ou délactosée et/ou délipidée.

**12.** - Procédés selon l'une des revendications 1 à 10, caractérisés par le fait que la matière traitée est à l'état deshydraté ou pulvérulent ou lyophilisé, puis remise en solution.

**13.** - Procédés selon l'une des revendications 1 à 12, caractérisés par le fait que la matière traitée est du colostrum.

**14.** - Procédés selon l'une des revendications 1 à 13, caractérisés par le fait que les différentes opérations sont effectuées à une température supérieure à 16,7°C en utilisant comme acide gras l'acide caprylique.

**15.** - Procédés selon l'une des revendications 1 à 14, caractérisés par le fait que les différentes opérations sont effectuées à la température ambiante.

**16.** - Procédés selon l'une des revendications 1 à 13, caractérisés par le fait que les différentes opérations sont effectuées à une température comprise entre 4°C et 8°C.

**17.** - Procédés selon la revendication 16 caractérisés par le fait que l'acide gras utilisé est l'acide hexanoïque.

**18.** - Procédés selon l'une des revendications 1 à 17 caractérisés par le fait que les ajustements de pH sont effectués à l'acide acétique pour baisser le pH et à la soude pour l'élever.

**19.** - Procédés selon l'une des revendications 1 à 18 caractérisés par le fait que la séparation de la fraction précipitée et de la fraction liquide est effectuée par microfiltration à un seuil de coupure de membrane compris entre 0,1 et 1 micromètre, préférentiellement 0,45 micromètre.

**20.** - Procédés selon l'une des revendications 1 à 19, caractérisés par le fait que l'agent utilisé pour la resolubilisation de la fraction précipitée récupérée après séparation, est la soude.

**21.** - Procédés selon l'une des revendications 1 à 20, caractérisés par le fait qu'ils comportent une étape terminale d'ultrafiltration de la ou des fractions récupérées, le seuil de coupure de la membrane d'ultrafiltration étant de 5 à 100 kD.

FIG.1

FIG.2

FIG.3

FIG.4

FIG.5

Office européen
des brevets

**RAPPORT DE RECHERCHE EUROPEENNE**

Numéro de la demande

EP    91 40 0236

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | EP-A-346217 (FONDATION NATIONAL DE TRANSFUSION SANGUINE)<br>* le document en entier * | 1-21 | C07K 3/24 |
| Y,D | EP-A-338229 (BIOTEST FARMA GMBH)<br>* page 3, ligne 35 - page 4, ligne 51 * | 1-21 | |
| A,D | EP-A-210109 (ASSOCIATION POUR LA RECHERCHE ET LE DEVELOPPEMENT DES METHODES ET PROC)<br>* le document en entier * | 1-21 | |
| A,D | CHEMICAL ABSTRACTS, vol. 100, no. 21, 21 mai 1984<br>Columbus, Ohio, USA<br>Habeeb, A. et al: "Preparation of human immunoglobulin by caprilic acid precipitation"<br>page 467; colonne 1; ref. no. 172769<br>* abrégé * | 1-21 | |
| A,D | CHEMICAL ABSTRACTS, vol. 73, no. 5, 03 août 1970<br>Columbus, Ohio, USA<br>Steinbuch M. et al: "Isolation of IgG immunoglobulin from human plasma using caprylic acid"<br>page 196; colonne 1-2; ref. no. 23460<br>* abrégé * | 1-21 | **DOMAINES TECHNIQUES RECHERCHES (Int. Cl.5)**<br><br>A61K<br>A23J<br>C07K |

Le présent rapport a été établi pour toutes les revendications

| Lieu de la recherche | Date d'achèvement de la recherche | Examinateur |
|---|---|---|
| LA HAYE | 02 MAI 1991 | FERNANDEZ Y BRA F. |

EPO FORM 1503 03.82 (P0402)